# EUROPÄISCHE PATENTSCHRIFT

(11) **EP 0 572 435 B1**
(45) Veröffentlichungstag und Bekanntmachung des Hinweises auf die Patenterteilung: **10.05.1995**
(21) Anmeldenummer: 92903585.5
(22) Anmeldetag: 25.01.1992
(51) Int. Cl.: A61B 17/36, B23K 26/02

(54) **VERFAHREN UND VORRICHTUNG ZUR ÜBERWACHUNG DER MATERIALBEARBEITUNG MITTELS GEPULSTEM LASERLICHT**
METHOD AND DEVICE FOR MONITORING TISSUE TREATMENT BY MEANS OF PULSED LASER LIGHT
PROCEDE ET DISPOSITIF POUR LA SURVEILLANCE DU TRAITEMENT TISSULAIRE AU MOYEN DE LUMIERE LASER EN REGIME PULSE

(30) Priorität: 19.02.1991 DE 4105060
(43) Veröffentlichungstag der Anmeldung: 08.12.1993
(73) Patentinhaber: MEDIZINISCHES LAZERZENTRUM LUBECK GMBH, D-23562 Lübeck (DE)
(72) Erfinder: SCHEU, Manfred, D-6761 Bisterschied (DE); ENGELHARDT, Ralf, D-3162 Uetze (DE); WETZEL, Wolfgang, D-2300 Kiel (DE)
(74) Vertreter: Vollmann, Heiko, Dipl.-Ing.
(86) Internationale Anmeldenummer: DE9200054
(87) Internationale Veröffentlichungsnummer: WO9214415

(56) Entgegenhaltungen:
- EP-A- 0 194 856
- EP-A- 0 312 650
- DE-A- 3 903 860

## Beschreibung

Die Erfindung betrifft ein Verfahren zur Überwachung der Materialbearbeitung mittels gepulstem Laserlicht, bei dem das Laserlicht über einen Lichtleiter an das Material herangeführt und reflektiertes Licht einer Auswerteinheit zugeführt wird, welche die Intensität des reflektierten Lichtes in zeitlicher Relation zum Laserpuls erfaßt und zur Bestimmung einer charakteristischen Materialeigenschaft auswertet. Weiterhin betrifft die Erfindung eine Vorrichtung zur Ausführung dieses Verfahrens gemäß den im Oberbegriff des Anspruchs 17 angegebenen Merkmalen.

Aus der EP-A-0 195 375 ist ein Verfahren bekannt, bei dem während der Bearbeitung von Gewebe mittels eines Bearbeitungslasers der Bearbeitungsbereich von einem Pilotlaser geringer Leistung bestrahlt wird, wobei das vom bearbeiteten Gewebe reflektierte Licht bei mehreren Wellenlängen ausgewertet wird, um festzustellen, ob das vom Bearbeitungslaser abzutragende Material Gewebe oder anderes Material ist. Das Verfahren dient dazu, Materialunterschiede zu erkennen, um einen Abtrag in nicht erwünschten Gebieten zu vermeiden. Das Verfahren nutzt die von unterschiedlichen Materialien ausgehenden unterschiedlichen Reflektionseigenschaften aus. Es ist jedoch für zahlreiche Anwendungsfälle nicht geeignet.

Bei dem Verfahren und der Vorrichtung zur Materialbearbeitung mittels Laser gemäß EP-A-0 312 650 wird der zeitliche Verlauf des zurückreflektierten Lichtes detektiert und mittels Kurvendiskriminator oder Schwellwertdetektoren dahingehend ausgewertet, daß der Bearbeitungspuls, sofern er auf nicht zu bearbeitendes Gewebe gerichtet ist, noch vor Erreichen seiner Maximalintensität abgebrochen werden kann. Die Auswertung des reflektierten Lichtes erfolgt somit im Verlauf der Entstehung der Gas- oder Plasmablase.

In der DE-PS 37 33 489 ist ein Verfahren beschrieben bei dem ein gepulster Laser als Bearbeitungslaser eingesetzt wird. Das gepulste Laserlicht wird über einen Lichtleiter dem Bearbeitungsmaterial zugeführt. Über diesen Lichtleiter wird vom bearbeiteten Material reflektiertes Laserlicht aufgenommen und einer Auswerteinheit zugeführt, welche die zeitliche Intensitätsverteilung des Lichtes bezogen auf den zeitlichen Verlauf des Laserpulses auswertet. Auch durch dieses Verfahren werden bestimmte charakteristische Materialeigenschaften detektiert, wie sie beispielsweise bei der Zerstörung von Harn- oder Gallensteinen erforderlich sind, um zu erkennen, ob der Bearbeitungslaser auf Gewebe oder Stein stößt.

Beim letztgenannten Verfahren können ebenfalls nur bestimmte Materialien voneinander unterschieden werden,und bei bestimmten Anwendungen hat sich dieses Verfahren als nicht brauchbar erwiesen. Das Verfahren ist insbesondere dann geeignet, wenn fluoreszierende Materialien unterschieden werden sollen. Das Verfahren ist eben im wesentlichen für die Steinzertrümmerung entwickelt und ausgebildet worden, da Harn- oder Gallensteine bei der Bearbeitung mit Laserlicht stark fluoreszieren.

Ausgehend vom letztgenannten Stand der Technik liegt der Erfindung die Aufgabe zugrunde, ein gattungsegemäßes Verfahren so auszubilden, daß die Bearbeitungsüberwachung unabhängig von der Leistung des Bearbeitungalasers ermöglicht wird. Insbesondere soll das Verfahren zur Detektion des Kammerswassers bei der ab externo Sklerostomie mittels gepulstem Laserlicht dienen. Des weiteren soll eine derartige Vorrichtung, insbesondere zur Ausführung des Verfahrens, geschaffen werden.

Der verfahrensmäßige Aufgabenteil wird bei einem gattungsgemäßen Verfahren durch die im kennzeichnenden Teil des Anspruchs 1 aufgeführten Merkmale gelöst. Der vorrichtungsmäßige Teil der Aufgabe wird durch die in Anspruch 17 aufgeführten Merkmale gelöst. Vorteilhafte Weiterbildungen der Erfindung sind durch die in den Unteransprüchen 2 bis 16 sowie 18 aufgeführten Merkmale gekennzeichnet.

Das erfindungsgemäße Verfahren ermöglicht die Überwachung der Materialbearbeitung mittels gepulstem Laserlicht bei voller Laserleistung. Das Verfahren eignet sich insbesondere zur Überwachung von Bearbeitungen im Grenzschichtbereich zwischen Gewebe und Flüssigkeit, Flüssigkeiten und Metallen unterschiedlicher Dichte, Pasten, Gels und dergleichen. Das Verfahren beruht auf der Erkenntnis, daß sich das unmittelbar vor dem Lichtleiterende befindliche Material, das während des Laserpulses verdampft wird, eine Gas- oder Plasmablase vor dem Lichtleiterende bildet. Diese Blase zerfällt nach einer gewissen Zeit und wird durch neue Flüssigkeit oder neues Material ersetzt. Die Zeit, in der eine solche Blase zerfällt und wieder durch Material ersetzt ist, stellt ein charakteristisches Materialmerkmal dar. Die dabei auftretenden Reflexionsänderungen sind deutlich zu unterscheiden, daher gut detektierbar bzw. auswertbar.

Mit dem erfindungsgemäßen Verfahren bzw. der entsprechenden Vorrichtung läßt sich insbesondere die ab externo Sklerostomie sehr exakt kontrollieren und somit eine Schädigung von Iris, Linsenkapsel oder Linse sicher vermeiden. Der Bearbeitungslaser kann hierdurch so gesteuert werden, daß bei Eintritt des Faserendes in die Augenkammer ein Signal ausgelöst wird, das den weiteren Bearbeitungsvorgang selbsttätig oder auch durch manuellen Eingriff beendet.

Als vom Laser unabhängige Lichtquelle wird vorzugsweise eine kontinuierliche Meßlichtquelle, bevorzugt ein weiterer Laser eingesetzt. Die Meßlichtquelle kann auch durch die Blitzlampe des Bearbeitungslasers gebildet sein, deren Leuchtdauer regelmäßig deutlich länger als die des Laserpulses ist, so daß das davon ausgehende Licht zur Reflexion am Lichtleiterende und zur späteren Auswertung eingesetzt wird. Hierdurch kann der konstruktive Aufwand der Vorrichtung vermindert werden.

Erfindungsgemäß kann das zur Auswertung erforderliche reflektierte Meßlicht nicht nur direkt über den Strahlengang des Lichtleiters zur Auswerteinheit geführt und dort gemessen werden, sondern statt dessen auch am Umfang des Lichtleiters gemessen werden.

Die Erfindung ist nachfolgend anhand von in den Zeichnungen dargestellten Ausführungsbeispielen näher erläutert. Es zeigen:
- Figur 1: ein Blockschaltbild der erfindungsgemäßen Vorrichtung,
- Figur 2: ein Diagramm über den zeitlichen Verlauf des reflektierten Lichtes,
- Figur 3: ein Diagramm über den zeitlichen Verlauf des reflektierten Lichtes bei einer ab externo Lasersklerostomie mit einem Ho-YAG-Laser.

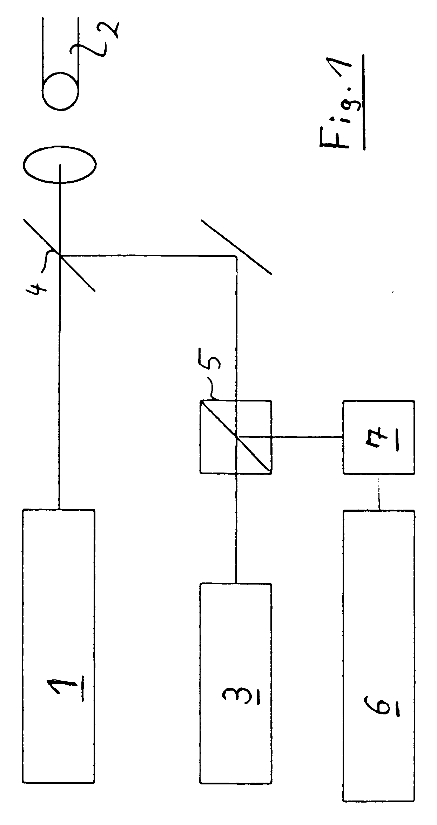

Das Blockschaltbild nach Figur 1 zeigt einen Bearbeitungslaser 1. Es handelt sich hierbei um einen gepulsten Laser. Dessen Bearbeitungslaserlicht wird in einen Lichtleiter 2 in Form einer Glasfaser eingekoppelt. Bei der dargestellten Ausführung arbeitet der Bearbeitungslaser 1 im Infrarotbereich, so daß schon zur optischen Kontrolle des Arbeitsergebnisses eine sichtbares Licht abgebende Bleuchtungsquelle 3 erforderlich ist. Hierzu ist ein Pilotlaser 3 vorgesehen, dessen Licht über einen teilreflektierenden Spiegel 4 in den Lichtleiter 2 eingekoppelt ist.

Das vom Pilotlaser, der in diesem Fall auch als Meßlichtquelle eingesetzt wird, abgegebene und am Ende des Lichtleiters 2 reflektierte Licht läuft in diesen zurück und wird über den teilreflektierenden Spiegel 4 sowie einen Polarisationsstrahlteiler 5 einer Auswerteinheit 6 zugeführt, die den Bearbeitungslaser 1 steuert oder zumindest zur Abgabe eines Signals zur Steuerung dieses Lasers vorgesehen ist.

Die Auswerteinheit 6 arbeitet elektronisch und wird durch das Ausgangssignal einer Fotodiode 7 angesteuert, auf die da reflektierte Meßlicht des Pilotlasert 3 auftrifft. Das über die Fotodiode gemessene elektrische Signal verkörpert die Intensität des auf die Fotodiode 7 auftreffenden Lichtes.

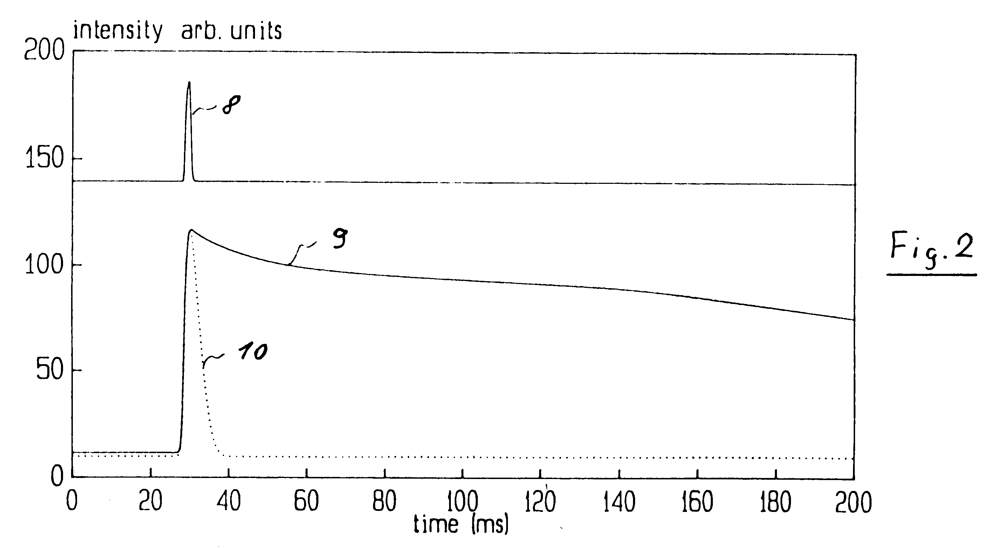

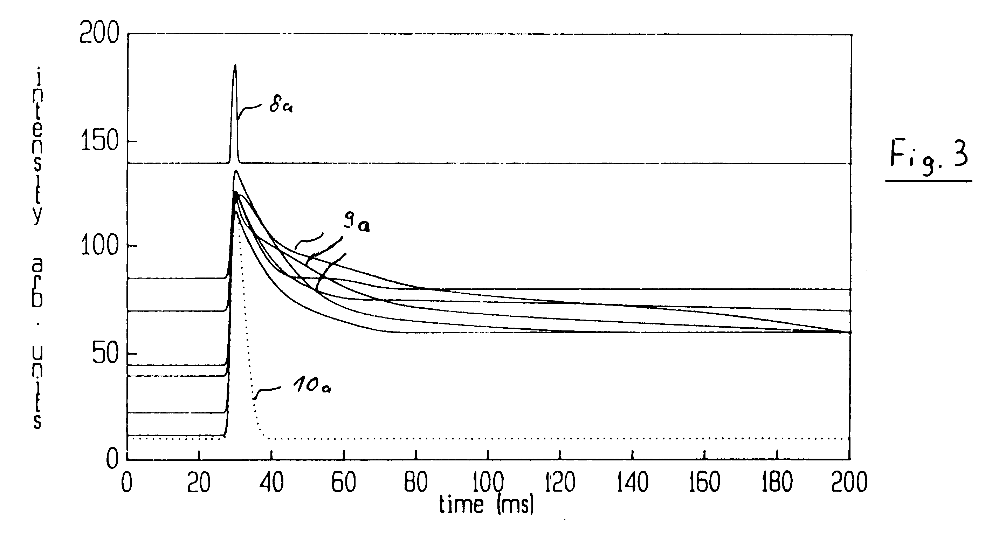

Derartige Signale sind durch die in den Diagrammen nach den Figuren 2 und 3 dargestellten unteren Kurven dargestellt. Die oberen Kurven sind nur zeitlich von quantitativer Aussagekraft, sie stellen den Bearbeitungslaserpuls zeitlich dar.

In Figur 2 sind im unteren Teil des Diagrammes zwei charakteristische Ausgangssignale des Detektors 7 dargestellt. Die horizontale Achse des Diagrammes stellt die Zeit dar, während die vertikale Achse die Intensität des reflektierten Lichtes angibt. Die mit 8 gekennzeichnete Kurve dient nur zur zeitlichen Orientierung und verdeutlicht den Bearbeitungslaserpuls. Die mit 9 gekennzeichnete Kurve ist charakteristisch für das Ausgangssignal des Detektors bei Kontakt des Lichtleiters 2 mit Gewebematerial, beispielsweise der Sklera. Diese Kurve zeigt zunächst einen konstanten flachen Verlauf, der die Reflexion des vom Pilotlaser ausgehenden Lichtes am Lichtleiterende darstellt. Während und nach dem Bearbeitungslaserpuls steigt das reflektierte Licht sehr steil an, um dann kontinuierlich und flach wieder abzufallen. Dies ist ein Signalverlauf, der typisch für eine Bearbeitung im Gewebe ist, da die sich vor dem Lichtleiterende bildende Gasblase nur verhältnismäßig langsam durch Flüssigkeit (Gewebeflüssigkeit) ersetzt werden kann, die durch das Gewebe hindurchdringen muß.

Ist das Gewebe der Sklera durchbohrt, so liegt das freie Ende der Lichtleitfaser im Augerkammerwasser. Es ergibt sich dann die in Figur 2 mit 10 gekennzeichnete punktiert dargestellte Kurve. Auch hier wird durch den Bearbeitungslaserpuls schlagartig Flüssigkeit unter Bildung einer Gasblase verdampft, so daß die Reflexion des Meßlichtes nach Auslösung des Laserpulses steil ansteigt. Aufgrund des direkt am Bearbeitungsort zur Verfügung stehenden Wassers zerfällt diese Blase jedoch schnell wieder, das umgebende Wasser gelangt binnen kurzer Zeit (nach ca. 10 Millisekunden) wieder zur Stirnseite des Lichtleiters, so daß der Ausgangszustand (wie vor dem Laserpuls) erreicht wird. Die Auswerteinheit 6 erkennt die charakteristischen Kurvenunterschiede und gibt ein entsprechendes Signal ab. Beispielsweise kann in diesem Fall bei der Sklerostomie der Bearbeitungslaser abgestellt werden, weil der in der Sklera zu schaffende Kanal hergestellt ist.

In Figur 3 ist der zeitliche Verlauf aller während einer ab externo Lasersklerostomie mit einem Ho-YAG-Laser (200 Mikrosekunden) auf die Detektoreinheit 7 fallenden Signale dargestellt. Vor Applikation des Bearbeitungslaserpulses streuen die Ausgangssignale bei Kontakt von Sklera und Lichtleitfaser stark und sind nicht deutlich von dem Fall zu unterscheiden, bei dem sich die Lichtleitfaser in Kontakt mit Kammerwasser befindet. Nach Applikation des Laserpulses (vorzugsweise nach 20 bis 200 Millisekunden) ist eine eindeutige Materialunterscheidung möglich. Den in Figur 2 dargestellten Kurven entsprechen hier die Kurven 8a, 9a und 10a.

## Patentansprüche

1. Verfahren zur Überwachung der Materialbearbeitung mittels gepulstem Laserlicht, bei dem das Laserlicht über einen Lichtleiter an das Material herangeführt wird und reflektiertes Licht einer Auswerteinheit zugeführt wird, welche die Intensität des reflektierten Lichtes in zeitlicher Relation zum Bearbeitungslaserpuls erfaßt und zur Bestimmung einer charakteristischen Materialeigenschaft auswertet, dadurch gekennzeichnet, daß auf das Material mindestens nach einem Bearbeitungslaserpuls Meßlicht durch den Lichtleiter hindurch gerichtet wird und daß die Auswertung des reflektierten Meßlichtes unmittelbar nach dem Bearbeitungslaserpuls derart erfolgt, daß der zeitliches Verlauf des Zerfalles einer sich durch den Bearbeitungslaserpuls im Material bildenden Gasblase als charakteristische Materialeigenschaft ermittelt wird, und daß anhand dieses zeitlichen Verlaufs eine Bestimmung des durch diesen Bearbeitungslaserpulses bearbeiteten Materiales erfolgt.

2. Verfahren nach Anspruch 1, dadurch gekennzeichnet, daß der Verlauf der durch den Bearbeitungslaser beim letzten Puls verursachten Änderung des Brechungsindexes des das distale Ende des Lichtleiters umgebenden Mediums detektiert wird.

3. Verfahren nach einem der vorhergehenden Ansprüche, dadurch gekennzeichnet, daß zur Erzeugung des Meßlichtes eine kontinuierliche Lichtquelle eingesetzt wird.

4. Verfahren nach einem der vorhergehenden Ansprüche, dadurch gekennzeichnet, daß als Meßlichtquelle ein Laser eingesetzt wird, vorzugsweise ein Helium-Neon-Laser oder eine Laserdiode.

5. Verfahren nach einem der vorhergehenden Ansprüche, dadurch gekennzeichnet, daß als Meßlichtquelle die Blitzlampe des Bearbeitungslasers verwendet wird.

6. Verfahren nach einem der vorhergehenden Ansprüche, dadurch gekennzeichnet, daß als Meßlichtquelle die Fluoreszenz des Bearbeitungslasers verwendet wird.

7. Verfahren nach einem der vorhergehenden Ansprüche, dadurch gekennzeichnet, daß die Meßlichtquelle polarisiertes Licht aussendet.

8. Verfahren nach einem der vorhergehenden Ansprüche, dadurch gekennzeichnet, daß das reflektierte Meßlicht in unterschiedlichen Wellenlängenbereichen erfaßt wird.

9. Verfahren nach einem der vorhergehenden Ansprüche, dadurch gekennzeichnet, daß ein Teil des innerhalb des Lichtleiters zurücklaufenden Meßlichtes über einen polarisierenden Strahlteiler, einen teilreflektierenden Spiegel oder eine Glasplatte etwa unter Brewsterwinkel einer Lichtmeßeinheit zugeführt wird.

10. Verfahren nach einem der vorhergehenden Ansprüche, dadurch gekennzeichnet, daß die Bestimmung der Intensität des reflektierten Meßlichtes ohne zusätzliche Optik im Strahlengang an der Außenseite des Lichtleiters erfaßt wird.

11. Verfahren nach einem der vorhergehenden Ansprüche, dadurch gekennzeichnet, daß der Detektor zur Intensitätserfassung des reflektierten Meßlichtes in der Auswerteinheit für den Wellenlängenbereich besonders sensitiv ist, in dem das von der Meßlichtquelleausgesendete Licht liegt, und daß das Laserlicht des Bearbeitungslasers einen im wesentlichen anderen Wellenlängenbereich aufweist.

12. Verfahren nach einem der vorhergehenden Ansprüche, dadurch gekennzeichnet, daß zu einem oder mehreren Zeitpunkten nach Beendigung des Bearbeitungslaserpulses die Intensität des im Lichtleiter zurücklaufenden Meßlichtes detektiert wird.

13. Verfahren nach einem der vorhergehenden Ansprüche, dadurch gekennzeichnet, daß bei Detektion einer abweichenden Materialeigenschaft ein Signal zur Steuerung des Bearbeitungslasers ausgelöst wird.

14. Verfahren nach einem der vorhergehenden Ansprüche, dadurch gekennzeichnet, daß bei einer ab externo Sklerostomie mittels eines gepulsten Bearbeitungslasers die Fistulierung und somit der Kontakt der Lichtleiterendfläche mit dem Kammerwasser detektiert wird.

15. Verfahren nach einem der vorhergehenden Ansprüche, dadurch gekennzeichnet, daß ein Ho-YAG- oder ein Er-YAG-Laser als Bearbeitungslaser eingesetzt wird.

16. Verfahren nach einem der vorhergehenden Ansprüche, dadurch gekennzeichnet, daß als Bearbeitungslaser ein Laser mit einer Lichtemission im Bereich hoher Wasserabsorption eingesetzt wird, vorzugsweise mit einem Wellenlängenbereich, der kleiner als 400 nm oder größer als 1,4 »m ist.

17. Vorrichtung zur Überwachung der Materialbearbeitung mittels gepulstem Laserlicht, mit einem Bearbeitungslaser (1), dessen Laserlicht mittels eines Lichtleiters (2) an das Material herangeführt ist, und mit einer Auswerteinheit (6, 7), der reflektiertes Licht zugeführt ist, wobei die Auswerteinheit (6, 7) die Intensität des reflektierten Lichtes in zeitlicher Relation zum Bearbeitungslaserpuls (8; 8a) erfaßt und zur Bestimmung einer charakteristischen Materialeigenschaft auswertet, dadurch gekennzeichnet, daß eine Meßlichtquelle (3) vorgesehen ist, mit der auf das Material mindestens nach einem Bearbeitungslaserpuls (8; 8a) Meßlicht richtbar ist, und daß die Auswerteinheit zur Erfassung des reflektierten Meßlichtes (9, 10; 9a, 10a) unmittelbar nach dem Bearbeitungslaserpuls derart ausgebildet ist, daß der zeitliche Verlauf des Zerfalles einer sich durch den Bearbeitungslaserpuls im Material bildenden Gasblase als charakteristische Materialeigenschaft ermittelbar ist, und anhand dieses zeitlichen Verlaufs das gerade durch den Bearbeitungslaserpuls bearbeitete Material bestimmbar ist.

18. Vorrichtung nach Anspruch 17, gekennzeichnet durch Merkmale eines oder mehrerer der Ansprüche 2 bis 16.

## Claims

1. A method for monitoring the processing of a material, by means of pulsed laser light, in which the laser light is brought to the material via an optical conductor and reflected light is supplied to an evaluation unit, which picks up the intensity of the reflected light in temporal relation to the processing laser pulse and evaluates said intensity to determine a characteristic material property, characterised in that measuring light is directed through the optical conductor on to the material at least after a processing laser pulse and that the evaluation of the reflected measuring light takes place directly after the processing laser pulse such that the temporal progress of the decay of a gas bubble formed in the material by the processing laser pulse is ascertained as a characteristic material property, and that a determination of the material processed by this processing laser pulse takes place with the aid of this temporal progress.

2. A method according to claim 1, characterised in that the progress of the change in the refractive index of the medium surrounding the distal end of the optical conductor, caused by the processing laser during the last pulse, is detected.

3. A method according to one of the preceding claims, characterised in that a continuous light source is used for producing the measuring light.

4. A method according to one of the preceding claims, characterised in that a laser, preferably a helium-neon laser or a laser diode, is used as the measuring light source.

5. A method according to one of the preceding claims, characterised in that the flash lamp of the processing laser is utilised as the measuring light source.

6. A method according to one of the preceding claims, characterised in that the fluorescence of the processing laser is utilised as the measuring light source.

7. A method according to one of preceding claims, characterised in that the measuring light source emits polarised light.

8. A method according to one of the preceding claims, characterised in that the reflected measuring light is picked up in various wavelength ranges.

9. A method according to one of the preceding claims, characterised in that a part of the measuring light returning within the optical conductor is supplied to a light measuring unit approximately at Brewster's angle via a polarising beam splitter, a partially reflecting mirror or a glass plate.

10. A method according to one of the preceding claims, characterised in that the determination of the intensity of the reflected measuring light is picked up with no additional optical system in the beam path on the outside of the optical conductor.

11. A method according to one of the preceding claims, characterised in that the detector for picking up the intensity of the reflected measuring light in the evaluation unit is particularly sensitive for the wavelength range in which the light emitted by the measuring light source lies, and that the laser light of the processing laser has a substantially different wavelength range.

12. A method according to one of the preceding claims, characterised in that at one or more instants after the end of the processing laser pulse the intensity of the measuring light returning in the optical conductor is detected.

13. A method according to one of the preceding claims, characterised in that on detection of a varying material property a signal for controlling the processing laser is triggered.

14. A method according to one of the preceding claims, characterised in that during an ab externo sclerotomy by means of a pulsed processing laser, fistulisation and also contact of the end surface of the optical conductor with the aqueous humour are detected.

15. A method according to one of the preceding claims, characterised in that a Ho-YAG laser or an Er-YAG laser is used as the processing laser.

16. A method according to one of the preceding claims, characterised in that a laser with a light emission in the range of high water-absorption, preferably with a wavelength range which is shorter than 400 nm or longer that 1.4 »m, is used as the processing laser.

17. A device for monitoring the processing of a material, by means of pulsed laser light, with a processing laser, the laser light of which is brought to the material by means of an optical conductor (2), and with an evaluation unit (6, 7) to which reflected light is supplied, in which the evaluation unit (6, 7) picks up the intensity of the reflected light is temporal relation to the processing laser pulse (8; 8a) and evaluates said intensity to determine a characteristic material properly, characterised in that a measuring light source (3) is provided, with which measuring light can be directed on to the material at least after a processing laser pulse (8; 8a), and that the evaluation unit, for picking up the reflected measuring light (9, 10; 9a, 10a) directly after the processing laser pulse, is constructed such that the temporal progress of the decay of a gas bubble formed in the material by the processing laser pulse is ascertained as a characteristic material property, and that the material just processed by the processing laser pulse can be determined with the aid of this temporal progress.

18. A device according to claim 17, characterised by the features of one or more of claims 2 to 16.

## Revendications

1. Procédé de surveillance de l'usinage d'un matériau, au moyen d'une lumière laser pulsée, dans lequel la lumière laser est dirigée sur le matériau par l'intermédiaire d'un guide de lumière et la lumière réfléchie est amenée à une unité d'évaluation qui appréhende l'intensité de la lumière réfléchie, selon une relation temporelle par rapport à l'impulsion du laser d'usinage et effectue une évaluation en vue de déterminer une propriété caractéristique du matériau, caractérisé en ce qu'est dirigé sur le matériau au moins une lumière de mesure, suite à une impulsion laser d'usinage, au moyen du guide de lumière, et en ce que l'évaluation de la lumière de mesure réfléchie s'effectue directement après une impulsion laser d'usinage, de manière que l'évolution temporelle de la désintégration d'une bulle de gaz en cours de formation dans le matériau, du fait de l'impulsion laser d'usinage, soit déterminée comme propriété caractéristique du matériau, et en ce que la caractérisation du matériau usiné par cette impulsion laser d'usinage s'effectue à l'aide de cette évolution temporelle.

2. Procédé selon la revendication 1, caractérisé en ce que l'évolution de la modification de l'indice de réfraction, provoquée par l'usinage laser lors de la dernière impulsion, du milieu entourant l'extrémité distale du guide de lumière est détectée.

3. Procédé selon l'une des revendications précédentes, caractérisé en ce qu'une source lumineuse continue est utilisée pour produire la lumière de mesure.

4. Procédé selon l'une des revendications précédentes, caractérisé en ce qu'est utilisée comme source lumineuse de mesure un laser, de préférence d'un laser hélium-néon, ou une diode laser.

5. Procédé selon l'une des revendications précédentes, caractérisé en ce qu'est utilisée comme source lumineuse de mesure la lampe à éclairs du laser d'usinage.

6. Procédé selon l'une des revendications précédentes, caractérisé en ce qu'est utilisée comme source lumineuse de mesure la fluorescence du laser d'usinage.

7. Procédé selon l'une des revendications précédentes, caractérisé en ce que la source lumineuse de mesure émet de la lumière polarisée.

8. Procédé selon l'une des revendications précédentes, caractérisé en ce que la lumière de mesure réfléchie est appréhendée dans différentes plages de longueur d'ondes.

9. Procédé selon l'une des revendications précédentes, caractérisé en ce qu'une partie de la lumière de mesure revenant à l'intérieur du guide de lumière est amenée, par l'intermédiaire d'un diviseur de rayons polarisant, d'un miroir à réflexion partielle ou d'une plaque de verre, à peu près sous l'angle de Brewster à une unité de mesure de lumière.

10. Procédé selon l'une des revendications précédentes, caractérisé en ce que la détermination de l'intensité de la lumière réfléchie est appréhendée sans optique supplémentaire dans le trajet du rayon, en face extérieure du guide de lumière.

11. Procédé selon l'une des revendications précédentes, caractérisé en ce que le détecteur destiné à appréhender l'intensité de la lumière de mesure réfléchie dans l'unité d'évaluation est particulièrement sensible à la plage de longueurs d'ondes dans laquelle se situe la lumière envoyée par la source lumineuse de mesure, et en ce que la lumière laser du laser d'usinage présente une plage de longueur d'ondes notablement différente.

12. Procédé selon l'une des revendications précédentes, caractérisé en ce que l'intensité de la lumière de mesure revenant dans le guide de lumière est détectée à un ou plusieurs instants, après achèvement de l'impulsion laser d'usinage.

13. Procédé selon l'une des revendications précédentes, caractérisé en ce qu'un signal de commande du laser d'usinage est déclenché lors de la détection d'une propriété du matériau traduisant un écart.

14. Procédé selon l'une des revendications précédentes, caractérisé en ce que, dans le cas d'une sclérostomie effectuée ab externo, au moyen d'un laser d'usinage pulsé, la fistulation et ainsi le contact de la surface d'extrémité du guide de lumière avec l'eau de la chambre est détectée.

15. Procédé selon l'une des revendications précédentes, caractérisé en ce qu'est utilisé comme laser d'usinage un laser Ho-YAG ou un laser Er-YAG.

16. Procédé selon l'une des revendications précédentes, caractérisé en ce qu'est utilisé comme laser d'usinage un laser avec une émission lumineuse dans la plage d'absorption élevée de l'eau, de préférence avec une plage de longueur d'ondes inférieure à 400 nm ou supérieure à 1,4 »m.

17. Dispositif de surveillance de l'usinage d'un matériau, au moyen d'une lumière laser pulsée, avec un laser d'usinage (1), dont la lumière laser est dirigée sur le matériau à l'aide d'un guide de lumière (2), et avec une unité d'évaluation (6, 7) à laquelle la lumière réfléchie est amenée, l'unité d'évaluation (6, 7) appréhendant l'intensité de la lumière réfléchie, selon une relation temporelle par rapport à l'impulsion du laser d'usinage (8; 8a) et effectuant une évaluation en vue de déterminer une propriété caractéristique du matériau, caractérisé en ce qu'est prévue une source lumineuse de mesure (3), à l'aide de laquelle au moins une lumière de mesure est susceptible d'être dirigée sur le matériau, suite à une impulsion laser d'usinage (8; 8a), et en ce que l'unité d'évaluation destinée à appréhender la lumière de mesure (9, 10; 9a, 10a) réfléchie directement après une impulsion laser d'usinage est réalisée de manière que l'évolution temporelle de la désintégration d'une bulle de gaz en cours de formation dans le matériau, du fait de l'impulsion laser d'usinage, puisse être déterminée à titre de propriété caractéristique du matériau et que le matériau justement usiné par l'impulsion laser d'usinage puisse être caractérisé à l'aide de cette évolution temporelle.

18. Dispositif selon la revendication 17, caractérisé par les caractéristiques de l'une ou plusieurs des revendications 2 à 16.
